# FASCICULE DE BREVET EUROPEEN

(11) **EP 2 337 511 B1**
(45) Date de publication et mention de la délivrance du brevet: **22.03.2017**
(21) Numéro de dépôt: 09753140.4
(22) Date de dépôt: 23.10.2009
(51) Int. Cl.: A61B 17/70

(54) **SYSTÈME D'OSTÉOSYNTHÈSE RACHIDIENNE**
WIRBELSÄULEN-OSTEOSYNTHESESYSTEM
SPINAL OSTEOSYNTHESIS SYSTEM

(30) Priorité: 23.10.2008 FR 0805881
(43) Date de publication de la demande: 29.06.2011
(73) Titulaire: Euros, 13600 La Ciotat (FR)
(72) Inventeur: Miladi, Lotfi, 92340 Bourg-la-Reine (FR)
(74) Mandataire: Orsini, Fabienne
(86) Numéro de dépôt international: PCT/FR2009/001246
(87) Numéro de publication internationale: WO 2010/046571

(56) Documents cités:
- WO-A-2007/060534
- FR-A- 2 795 623
- US-A1- 2006 241 594
- US-A1- 2007 161 994
- US-B1- 6 440 132

## Description

L'invention concerne un système d'ostéosynthèse rachidienne.

Les systèmes traditionnels d'ostéosynthèse rachidienne sont conçus pour réaliser une arthrodèse de la colonne et comprennent en général deux tiges rigides parallèles, fixées par des crochets ou des vis pédiculaires sur des vertèbres à réunir, et des éléments de liaison transversale entre les deux tiges, ces tiges étant immobilisées sur les crochets et les vis pédiculaires, en général par des vis de blocage.

Ces systèmes présentent un certain nombre d'inconvénients :
- ils forment des ensembles rigides et indéformables qui bloquent le dos et empêchent une flexion de la colonne vertébrale,
- lorsqu'ils sont implantés sur des enfants, ils s'opposent à la croissance de la colonne vertébrale,
- ils sont à l'origine d'une dégradation anatomique et fonctionnelle à long terme des zones adjacentes de la colonne laissées libres, en raison de leur surcharge de mobilité, ce qui cause des arthroses, des douleurs, etc..

On a déjà proposé des systèmes d'ostéosynthèse destinés à limiter au moins en partie certains de ces inconvénients, dans lesquels les tiges rigides sont montées de façon coulissante dans des supports (par exemple US 2006/0241594-A1), ces supports pouvant eux-mêmes être montés de façon pivotante sur des vis pédiculaires (par exemple EP 1 665 994-A1), ou dans lesquels les tiges rigides sont remplacées par une pluralité d'éléments articulés les uns au bout des autres et associés à des moyens de serrage permettant de les bloquer dans une position ou une orientation appropriée (par exemple FR 2 715 825-A1).

Toutefois, ces systèmes ont eux-mêmes leurs inconvénients :
- celui décrit dans EP 1 665 994-A1 est composé d'un grand nombre de pièces différentes et est relativement lourd et encombrant,
- celui décrit dans FR 2 715 825-A1 est rigide et indéformable quand il est implanté et s'oppose à la flexion de la colonne ainsi qu'à sa croissance.

Il est par ailleurs connu (WO 2004/010881) de fixer ces systèmes sur les vertèbres, non par des vis pédiculaires, mais par des liens souples qui relient une pièce support de tige à une côte ou une apophyse transverse.

On connaît par ailleurs du document US20060241594 un système d'ostéosynthèse comprenant deux tiges chacune fixée à la colonne vertébrale d'un patient par trois vis rachidiennes. Pour ne pas empêcher la croissance de la colonne vertébrale d'un jeune patient, chaque tige est fixée en son centre à l'une des trois vis rachidiennes. et est montée coulissante dans les deux autres vis rachidiennes.

Les vis rachidiennes conçues pour laisser la tige libre de coulisser comportent chacune un corps fileté et une tête en forme de U adaptée à recevoir la tige. Elles comportent également chacune un bouchon adapté à se visser dans la tête de vis pour empêcher la tige de sortir de son logement, mais qui ne vient pas s'appuyer sur la tige pour laisser cette dernière libre de coulisser.

L'inconvénient de ce système est que la tige n'est pas libre de pivoter par rapport au vis, ce qui génère des risques de coincement de la tige.

Le document US6440132 décrit une vis rachidienne dont la tête forme un berceau d'accueil d'une tige longitudinale. Dans ce document, la vis est équipée d'un bouchon adapté à refermer le berceau pour y bloquer la tige longitudinale.

Le document FR2795623 décrit un crochet rachidien dont la tête forme un berceau d'accueil d'une tige longitudinale. Dans ce document. le crochet est équipé d'un bouchon qui est encliqueté sur la tête du crochet de telle manière que ce bouchon reste libre de pivoter sur le crochet et qu'il bloque la tige longitudinale dans le berceau d'accueil.

Le document US2007/0161994 décrit une vis rachidienne qui comporte un corps fileté, une tête en forme de U qui délimite un logement d'accueil d'une rotule, et un loquet monté sur la tête via un axe de charnière de manière à pouvoir bloquer la rotule dans son logement grâce à une vis). La rotule est traversée par un alésage pour accueillir la tige.

Le document WO2007/060534 divulgue un système d'ostéosynthèse comprenant des vis rachidiennes dont les têtes sont équipées d'organes de connexion, et des moyens de liaison permettant de connecter entre eux les organes de connexion. Ces moyens de liaison comprennent des blocs élastiques qui s'interposent chacun entre deux organes de connexion, et un câble qui est adapté à traverser les organes de connexion pour comprimer les blocs entre les deux organes de connexion.

Le document US2003/0187439 décrit une vis rachidienne comportant une tête sphérique montée mobile en rotation dans un berceau d'accueil d'une tige longitudinale. Dans ce document, il est prévu un écrou qui vient se visser sur la face externe du berceau de manière à le comprimer pour simultanément bloquer la tige mobile par rapport au berceau et le berceau par rapport à la vis.

Le document US5176680 décrit une vis rachidienne dont la tête forme un berceau d'accueil d'une rotule traversée par une tige longitudinale. Un bouchon est alors prévu pour venir comprimer la rotule de telle sorte qu'une fois l'implant mis en place sur le patient, la tige longitudinale soit bloquée par rapport à la vis.

Le document US5486174 divulgue une vis rachidienne qui comporte un corps fileté et une tête annulaire qui délimite un logement d'accueil. d'une rotule traversée par une tige rigide. Sa tête annulaire présente un alésage taraudé qui accueille une vis permettant de verrouiller la rotule au cours de l'opération chirurgicale.

Le document US2007/0191843 divulgue une vis rachidienne dont la tête est mobile en rotation par rapport au corps fileté, autour d'un axe confondu avec l'axe du corps fileté. La tête est par ailleurs équipée de moyens de crochetage d'une tige longitudinale rigide.

Le document WO95/17863 divulgue un crochet rachidien dont la tête délimite un passage pour une tige longitudinale. Dans ce document, le crochet est équipé de manchons pour bloquer le coulissement de la tige longitudinale.

Le document US2002/0035366 décrit une vis rachidienne comportant une tête sphérique montée mobile en rotation dans un berceau d'accueil d'une tige longitudinale. Il est alors prévu un écrou qui vient se visser dans le berceau de manière à bloquer la tige mobile par rapport au berceau.

Le document US6547789 divulgue différents modes de réalisation d'un crochet rachidien dont la tête est prévue pour accueillir transversalement une tige longitudinale.

Le document US20080021459 décrit un système d'ostéosynthèse permettant de soigner un problème de dégénérescence discale. Ce système comprend une courte tige qui est fixée à deux vertèbres successives de la colonne vertébrale d'un patient par deux vis rachidiennes. Il comprend également un élément tampon enfilé sur la tige_{.} entre les deux vis rachidiennes.

L'une des vis rachidiennes permet d'attacher fixement la tige à la colonne, tandis que l'autre vis rachidienne laisse à la tige des mobilités de translation et de pivotement. Cette seconde vis rachidienne comporte à cet effet un corps fileté et une tête annulaire qui reçoit intérieurement une rotule.

Le document US2008195159 décrit une vis pédiculaire comprenant une tête en forme de berceau qui délimite un passage pour une tige longitudinale. Ce berceau accueille intérieurement des moyens pur le serrage et la fixation de la tige longitudinale.

La présente invention a notamment pour but de pallier les inconvénients précités des systèmes connus et d'éviter l'arthrodèse de la colonne grâce à un système d'ostéosynthèse rachidienne qui est plus simple, plus léger et moins encombrant que ceux de la technique antérieure et qui ne s'oppose pas à la mobilité ou à la flexibilité de la colonne ainsi qu'à sa croissance chez l'enfant.

Elle propose à cet effet un système d'ostéosynthèse rachidienne comprenant au moins une tige longitudinale reliée des moyens, tels que des vis pédiculaires ou des crochets, de fixation sur les vertèbres, caractérisé en ce que la tige est flexible et coulisse librement en position de service par rapport à au moins certains des moyens de fixation.

La flexibilité de la tige et son coulissement par rapport aux moyens de fixation aux vertèbres permettent au système de redresser la colonne et de se déformer sans exercer d'efforts sur les vertèbres pour suivre les flexions du dos, qui garde toute sa mobilité. Cela permet également la croissance de la colonne chez l'enfant, sans qu'il soit nécessaire de modifier le système implanté.

Selon une autre caractéristique de l'invention, la tige flexible est réalisée en une matière plastique biocompatible telle que le PEEK (polyéther éther cétone).

Cette matière est un thermoplastique semi-cristallin qui est biocompatible et implantable, stérilisable, qui a des caractéristiques mécaniques proches de celles des métaux et qui les conserve après implantation.

La flexibilité de cette tige, supérieure à celle d'une tige métallique de mêmes dimensions, contribue à préserver la mobilité du dos d'un patient auquel on a implanté le système selon l'invention.

Selon d'autres caractéristiques de l'invention, la tige est guidée en translation dans des organes de connexion aux moyens de fixation sur les vertèbres, et chaque organe de connexion comprend un corps annulaire traversé par la tige et des moyens de montage de ce corps sur un moyen de fixation précité.

Avantageusement, chaque organe de connexion est monté par encliquetage élastique sur un moyen de fixation précité et comprend deux pattes latérales se terminant par des moyens d'encliquetage dans des moyens complémentaires du moyen de fixation.

Cette caractéristique facilite beaucoup l'implantation du système qui consiste alors à monter tous les organes de connexion nécessaires sur la tige et à fixer ensuite ces organes de connexion par encliquetage sur des vis pédiculaires, par exemple, déjà fixées sur les vertèbres, ce qui ne serait pas possible si la tige était rigide.

En position de service (à l'état implanté), la tige peut pivoter, dans au moins certains des organes de connexion, autour d'un axe transversal perpendiculaire à l'axe de la tige.

En outre, dans chaque organe de connexion, la tige est de préférence immobilisée en rotation autour d'un axe perpendiculaire à la tige et au plan de la colonne, c'est-à-dire par exemple autour de l'axe d'une vis pédiculaire qui constitue un moyen de fixation sur une vertèbre.

L'invention prévoit encore des moyens de blocage en translation de la tige dans au moins un organe de connexion précité.

La tige peut être ainsi bloquée en translation à une extrémité ou entre ses extrémités sur un ou plusieurs des moyens de fixation aux vertèbres, en particulier pour maintenir une distance prédéterminée entre des vertèbres.

Selon une autre caractéristique de l'invention, le système comprend deux tiges flexibles parallèles reliées à des moyens de fixation sur les vertèbres et réunies entre elles par des liaisons transversales.

Ces liaisons transversales sont rigides et fixées sur certains des organes précités de connexion aux moyens de fixation.

Les moyens de fixation comprennent, comme indiqué plus haut, des vis pédiculaires ou des crochets, les crochets comprenant avantageusement des colliers de serrage en une matière flexible et biocompatible telle par exemple qu'une matière plastique du type PEEK, les colliers de serrage permettant une fixation sur les vertèbres au niveau des lames ou des apophyses transverses ou sur les côtes dans la région thoracique.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 représente schématiquement une colonne vertébrale comportant un système d'ostéosynthèse selon l'invention ;
- la figure 2 est une vue schématique en perspective éclatée d'un organe de fixation pédiculaire selon l'invention ;
- la figure 3 est une vue schématique en perspective de la vis de la figure 2 ;
- la figure 4 est une vue de dessus d'une embase de la vis ;
- la figure 5 est une vue schématique en perspective éclatée d'une variante de réalisation de l'invention;
- la figure 6 est une vue schématique en perspective du dispositif de la figure 5 ;
- la figure 7 représente schématiquement un moyen de fixation d'une liaison transversale à une autre tige flexible;
- la figure 8 est une vue en perspective d'un crochet de fixation.

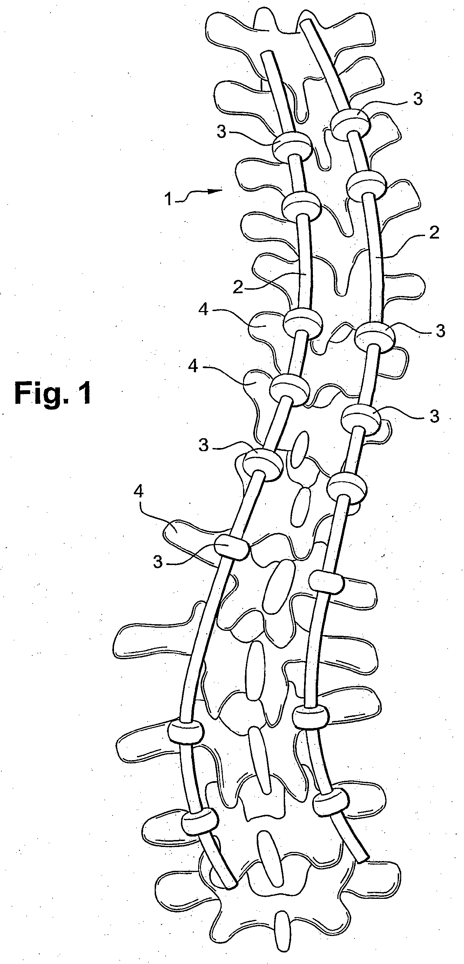

On a représenté schématiquement en figure 1 une colonne vertébrale 1 et un système d'ostéosynthèse selon l'invention, qui comprend essentiellement deux tiges longitudinales parallèles 2 guidées dans des organes 3 de connexion à des moyens de fixation sur les vertèbres 4.

Chaque tige 2 est montée fixement dans au moins un des organes 3 de connexion et peut coulisser et pivoter dans les autres organes de fixation, ce qui permet de conserver la mobilité du dos du patient et autorise, chez un enfant, la croissance de la colonne.

Chaque tige 2 est de plus flexible, ce qui lui permet de suivre les mouvements du dos tout en redressant la colonne.

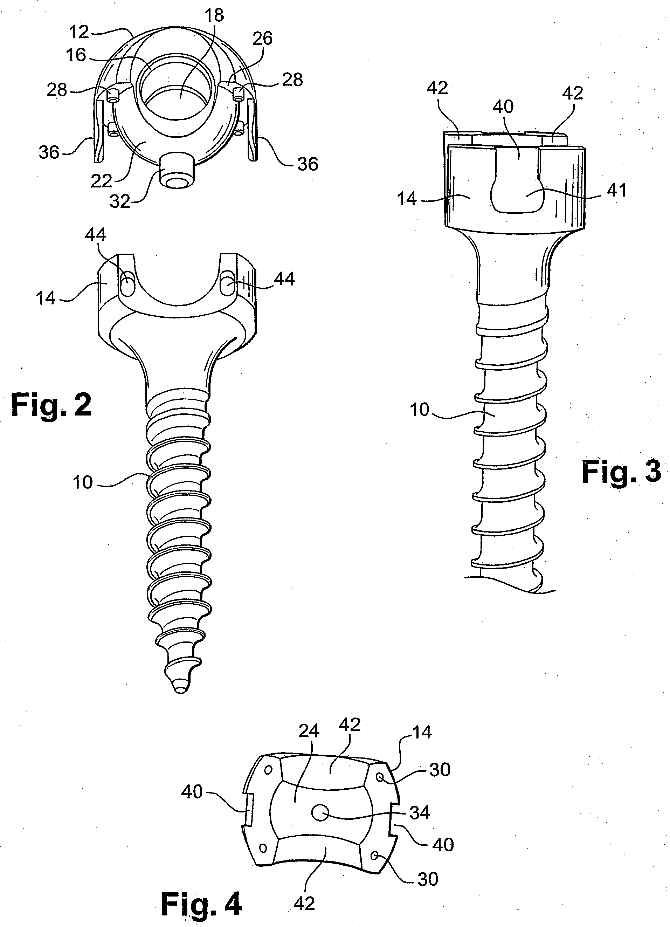

Les figures 2 à 4 représentent un moyen de fixation à vis pédiculaire selon l'invention, ce moyen comprenant une tige filetée 10 d'un type classique ou canulé (permettant son introduction sur une broche guide), réalisée en métal (par exemple en titane ou en acier inoxydable) et une embase 14 formée à une extrémité supérieure de la tige filetée 10, un organe de connexion 12 étant fixé sur l'embase 14 par clipsage ou encliquetage élastique.

L'organe 12 comporte un logement de réception et de guidage d'une rotule sphérique 16 traversée axialement par un alésage 18 dans lequel une tige cylindrique flexible 2 s'étend avec jeu de façon à pouvoir coulisser librement dans la rotule 16.

Dans ce mode de réalisation de l'invention, la partie inférieure semi annulaire 22 de l'organe 12 forme une surface 24 de guidage de la rotule 16 et est rapportée sur la partie supérieure de l'organe 12 et fixée à celle-ci par tout moyen approprié, par exemple par soudure ou par collage.

La base 26 de l'organe 12 comporte des moyens tels que des pions 28 de positionnement et de centrage sur l'embase 14 de la vis 10, qui sont reçus dans des petits trous 30 de la face supérieure de cette embase, un pion axial supplémentaire 32 étant formé sur la pièce semi-annulaire 22 et reçu dans un trou central 34 de la surface 24 de l'embase 14.

La fixation de l'organe 12 sur l'embase 14 est réalisée au moyen de deux pattes parallèles 36 formées aux extrémités d'un cavalier en U 38 monté dans une gorge semi-circulaire de la surface extérieure de l'organe 12, les deux pattes 36 étant destinées à s'engager dans deux cavités ou rainures latérales 40 de l'embase 14 et comportant à leurs extrémités des moyens d'encliquetage qui s'engagent élastiquement dans des moyens complémentaires prévus aux extrémités inférieures des rainures 40.

Par exemple, les extrémités des pattes 36 peuvent présenter un élargissement destiné à être reçu dans une extrémité élargie 41 des rainures latérales 40 de l'embase 14, comme représenté en figure 3.

Ainsi, il suffit de positionner l'organe de connexion 12 sur l'embase 14, en engageant les pions 28 et 32 dans les orifices 30 et 34 de l'embase, et de le pousser sur l'embase pour que les pattes 36 s'engagent et se bloquent dans les rainures 40.

L'organe 12 peut être séparé de l'embase 14 lorsqu'on écarte les extrémités des pattes 36 et qu'on exerce une traction sur l'organe 12 pour l'écarter de l'embase 14.

Comme on le voit en figure 4, la surface interne sphérique 24 de l'embase 14 se trouve entre deux surfaces tronconiques 42 qui vont en s'élargissant vers les faces externes de l'embase 14 pour permettre un débattement angulaire de la tige 2 par rapport à la vis 10.

De même, une surface interne sphérique de la partie supérieure de l'organe 12 est formée entre deux surfaces tronconiques correspondant aux surfaces 42 de l'embase 14.

Le débattement angulaire de la tige 2 autour de son centre de rotation dans l'organe de connexion 12 est par exemple de l'ordre de 15 à 35° dans toutes les directions. Préférentiellement, le débattement angulaire autour de l'axe de la vis 10 est restreint ou sensiblement nul.

Les faces externes plates de l'embase 14 comportent par ailleurs des empreintes ou renfoncements symétriques 44 servant à la fixation sur cette embase d'un outil de manipulation et d'insertion de la vis de fixation.

La réalisation représentée aux dessins a l'avantage que la rotule 16 de guidage de la tige 20 est prisonnière de l'organe de connexion 12 et ne peut s'en échapper.

La tige flexible 2 est réalisée en une matière biocompatible, telle de préférence qu'une matière plastique du type PEEK, à laquelle on peut donner une forme cintrée appropriée par moulage ou par thermoformage. Le PEEK a un module de flexion de 4 GPa.

Cette tige 2 a une diamètre compris entre 4 et 6 mm par exemple et peut avoir une section circulaire sur toute sa longueur ou bien non circulaire sur tout ou partie de sa longueur, auquel cas elle est guidée dans un passage 18 de forme correspondante de la rotule 16 qui permet à la tige 2 de coulisser librement en direction axiale mais qui l'empêche de tourner autour de son axe, et qui s'oppose donc à une torsion ou un vrillage de la tige 2.

On peut par ailleurs utiliser des tiges 2 de diamètres différents et de flexibilités différentes en fonction des implantations à réaliser.

Dans une variante de réalisation, la rotule sphérique 16 est remplacé par un organe de guidage cylindrique, comportant comme la rotule 16 un alésage ou passage traversant de guidage en translation de la tige 2, l'axe de l'organe de guidage cylindrique étant perpendiculaire à l'axe de cette tige et cet organe cylindrique étant monté de façon à pivoter autour d'un axe transversal qui est perpendiculaire à l'axe de la tige filetée 10 et à l'axe de la tige 2, c'est-à-dire un axe qui s'étend approximativement entre les deux pattes 36 d'encliquetage élastique de l'organe de connexion 12.

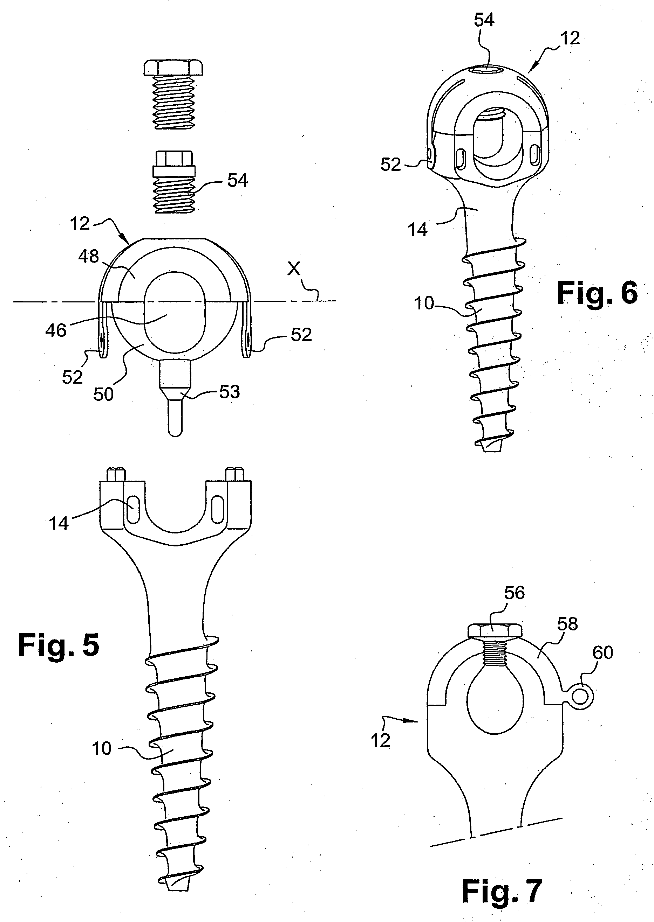

Les figures 5 et 6 représentent un autre mode de réalisation de l'invention, qui diffère de celui précédemment décrit en ce que l'organe de connexion 12 ne comprend pas de rotule 16, cet organe étant constitué essentiellement d'un corps annulaire présentant une ouverture centrale 46 de forme oblongue traversée par la tige flexible 2 qui peut coulisser librement dans cette ouverture le long de son axe et pivoter autour d'un axe transversal X perpendiculaire à l'axe de la tige 2 et à l'axe de la vis 10.

De préférence, le pivotement de la tige 2 dans l'ouverture 46 autour de l'axe de la vis 10 est nul ou sensiblement nul. Il suffit pour cela que la largeur de l'ouverture 46 le long de l'axe transversal X soit légèrement supérieure au diamètre de la tige 2.

L'organe de connexion 12 est constitué de deux pièces semi-annulaires 48, 50, qui sont fixées l'une à l'autre par soudage laser ou soudage TIG et forment un corps annulaire que l'on fixe sur l'embase 14 de la vis 10 par clipsage ou encliquetage élastique au moyen de pattes latérales 52, ces pattes étant rapportées sur l'organe 12 ou formées d'une pièce avec celui-ci.

Dans cette réalisation, des picots de positionnement de l'organe 12 sont formés en saillie sur la face supérieure de l'embase 14 et reçus dans des trous correspondants de la face inférieure de l'organe 12, et un doigt axial est formé en saillie sur la partie inférieure 50 de cet organe et est reçu dans un passage central de l'embase 14 pour le centrage et le guidage de l'organe 12.

La partie supérieure 48 de l'organe 12, du côté opposé à la vis 10, comporte un orifice taraudé qui s'étend dans l'axe de la vis 10 et qui reçoit une vis 54 de blocage de la tige flexible 2 dans l'organe de connexion 12, cette vis pouvant aussi former un moyen de limitation du pivotement de la tige 2 autour de l'axe transversal X.

On peut aussi, au moyen d'une autre vis 56 engagée dans l'orifice taraudé, fixer sur l'organe de connexion 12 des moyens de liaison transversale à une autre tige flexible 2, comme représenté en figure 7.

Ces moyens de liaison transversale comprennent une pièce semi-annulaire 58 fixée sur la partie supérieure 48 de l'organe 12 par la vis 56, cette pièce 58 comprenant à une extrémité une oreille latérale 60 formée avec un trou central pour l'attache d'une barre transversale de liaison à une autre tige flexible 2.

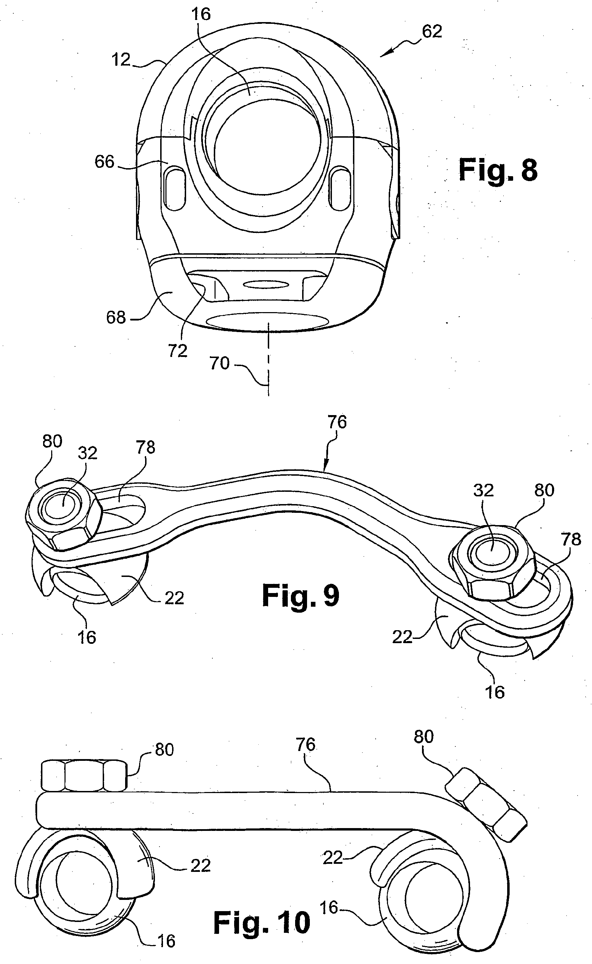

La figure 8 représente un crochet 62 de fixation sur une vertèbre ou sur une côte, comprenant un organe de connexion 12 identique à celui déjà décrit, qui se fixe par encliquetage élastique sur une embase 66 dont la partie inférieure 68 est rotative autour d'un axe central 70 sensiblement vertical sur le dessin. La partie inférieure 68 de l'embase comporte une fente 72 de passage d'un lien souple, par exemple en polymère tel que du PEEK, du type collier de serrage, qui permet de fixer le crochet 62 sur une apophyse d'une vertèbre ou sur une côte de la cage thoracique. On peut en particulier fixer les crochets 62 sur des vertèbres cervicales, ce qui évite d'implanter des vis dans ces vertèbres et de risquer de provoquer une usure ou une lyse de l'os.

## Revendications

1. Système d'ostéosynthèse rachidienne, comprenant :
- au moins une tige longitudinale (2) flexible en matière plastique biocompatible,
- des moyens (10) de fixation adaptés à être fixés sur des vertèbres,
- des organes (12) de connexion qui sont chacun reliés à un desdits moyens (10) de fixation, et qui sont adaptés à guider ladite tige longitudinale (2),
des premiers organes (12) de connexion étant conformés pour, en position de fonction, laisser la tige longitudinale (2) libre de coulisser et libre de pivoter autour d'au moins un axe transversal (X) perpendiculaire à l'axe de la tige, chaque premier organe (12) délimitant un logement de réception et de guidage_{.}
un second desdits organes (12) de connexion comprenant des moyens (54) de blocage en coulissement de la tige (2),
**caractérisé en ce**
**qu'**il est prévu une rotule sphérique (16) qui est engagée dans le logement de réception et de guidage délimité par le premier organe (12) et qui est traversée axialement par un alésage (18) dans lequel s'étend la tige longitudinale (2) avec jeu, et
en ce que les premiers organes de connexion (12) sont montés de façon amovible sur les moyens de fixation (10, 62), et
en ce que les organes de connexion (12) sont fixés par encliguetage élastique sur les moyens de fixation (10, 62) et comprennent des pattes latérales (36) se terminant par des moyens d'encliquetage destinés à être engagés dans des moyens complémentaires (40, 41) des moyens de fixation (10, 62).

2. Système selon la revendication 1, **caractérisé en ce que**, dans ledit second organe de connexion (12), la tige est immobilisée en rotation autour d'un axe perpendiculaire à l'axe de la tige et à l'axe transversal (X) précité.

3. Système selon l'une des revendications 1 et 2, **caractérisé en ce que** chaque organe de connexion (12) comporte des moyens (28, 32) de positionnement et de centrage sur une embase (14) d'un moyen (10, 62) de fixation.

4. Système selon l'une des revendications précédentes, **caractérisé en ce que** la tige (2) est en une matière plastique biocompatible telle que le PEEK.

5. Système selon l'une des revendications 1 à 4, **caractérisé en ce que** la tige (2) est au moins en partie à section non circulaire et **en ce que** chaque organe de connexion (12) comporte un passage de section correspondante traversé avec jeu par la tige et s'opposant à une torsion de celle-ci.

6. Système selon l'une des revendications 1 à 5, **caractérisé en ce qu'**il comprend deux tiges flexibles longitudinales parallèles (2) reliées à des moyens (10) de fixation sur les vertèbres et réunies entre elles par des liaisons transversales (76) fixées à leurs extrémités sur des organes (12) de connexion montés sur les tiges flexibles.

7. Système selon l'une des revendications précédentes, **caractérisé en ce que** les moyens de fixation sur les vertèbres comprennent des vis pédiculaires (10) ou des crochets (42) à colliers de serrage réalisés en une matière flexible et biocompatible telle que du PEEK.

8. Système selon l'une des revendications précédentes, **caractérisé en ce que** les moyens de blocage comprennent une vis (54) vissé dans un orifice taraudé dudit second organe (12).

## Patentansprüche

1. Wirbelsäulen-Osteosynthese-System mit
- wenigstens einem länglichen flexiblen Stab (2) aus biokompatiblem Plastikmaterial,
- Befestigungsmitteln (10), die dazu ausgelegt sind, an Wirbeln befestigt zu werden,
- Verbindungsorganen (12), von denen jedes mit einem der Befestigungsmittel (10) verbunden ist und die dazu ausgelegt sind, den länglichen Stab (2) zu führen,
wobei erste Verbindungsorgane (12) so ausgestaltet sind, daß sie in der Betriebsstellung den länglichen Stab (2) frei gleiten und sich frei um wenigstens eine zur Achse des Stabs senkrechte Querachse (X) schwenken lassen, wobei jedes erste Organ (12) einen Aufnahme-und Führungsraum begrenzt,
wobei ein zweites der Verbindungsorgane (12) Mittel (54) zum Blockieren des Gleitens des Stabs (2) aufweist,
**dadurch gekennzeichnet,**
**daß** ein Kugelkopf (16) vorgesehen ist, der in dem vom ersten Organ (12) begrenzten Aufnahme- und Führungsraum gelagert ist und der axial von einer Bohrung (18) durchquert ist, in der sich der längliche Stab (2) mit Spiel erstreckt,
**daß** die ersten Verbindungsorgane (12) auf den Befestigungsmitteln (10, 62) abnehmbar befestigt sind und
**daß** die Verbindungsorgane (12) durch elastisches Einrasten in den Befestigungsmitteln (10, 62) befestigt sind und seitliche Arme (36) aufweisen, an deren Enden sich Einrastmittel befinden, die dazu bestimmt sind, in komplementäre Mittel (40, 41) der Befestigungsmittel (10, 62) eingebracht zu werden.

2. System gemäß Anspruch 1, **dadurch gekennzeichnet, daß** der Stab im zweiten Verbindungsorgan (12) gegen eine Drehung um eine zur Achse des Stabes und zur vorgenannten Querachse (X) senkrechte Achse gesichert ist.

3. System gemäß einem der Ansprüche 1 und 2, **dadurch gekennzeichnet, daß** jedes Verbindungsorgan (12) Mittel (28, 32) zum Positionieren und zum Zentrieren auf einem Sockel (14) eines Befestigungsmittels (10, 62) aufweist.

4. System gemäß einem der vorangehenden Ansprüche, **dadurch gekennzeichnet, daß** der Stab (2) aus einem biokompatiblen Plastikmaterial wie zum Beispiel PEEK ist.

5. System gemäß einem der Ansprüche 1 bis 4, **dadurch gekennzeichnet, daß** der Stab (2) wenigstens teilweise einen nicht kreisförmigen Querschnitt aufweist und daß jedes Verbindungsorgan (12) einen Durchgang mit entsprechendem Querschnitt aufweist, der vom Stab mit Spiel durchquert wird und sich einer Drehung desselben widersetzt.

6. System gemäß einem der Ansprüche 1 bis 5, **dadurch gekennzeichnet, daß** es zwei längliche flexible parallele Stäbe (2) aufweist, die miteinander durch Querverbindungen (76) verbunden sind, die an deren Enden an an den flexiblen Stäben angebrachten Verbindungsorganen (12) befestigt sind.

7. System gemäß einem der vorangehenden Ansprüche, **dadurch gekennzeichnet, daß** die Mittel zum Befestigen an den Wirbeln Pedikelschrauben (10) oder Haken (42) mit Klemmringen aufweisen, die aus einem flexiblen und biokompatiblen Material wie zum Beispiel PEEK sind.

8. System gemäß einem der vorangehenden Ansprüche, **dadurch gekennzeichnet, daß** die Blockiermittel eine in ein Gewindeloch des besagten zweiten Organs (12) eingedrehte Schraube (54) aufweisen.

## Claims

1. A spinal osteosynthesis system comprising :
- at least one flexible longitudinal rod (2) made of a biocompatible plastics material,
- fastening means (10) suitable to be fastened onto vertebrae,
- connection members (12) suitable to guide said longitudinal rod (2), each connection member (12) being connected to one of said fastening means (10),
first connection members (12) being adapted to let, in an operating position, the longitudinal rod (2) free to slide and to pivot about at least one transverse axis (X) that is perpendicular to the axis of the rod, each first connection member (12) including a receiving and guiding housing,
one second connection member (12) comprising blocking means (54) to block the sliding of the rod (2),
the system being **characterized in that** it comprises a spherical ball (16) that is engaged into said housing and that is axially crossed by a bore (18) in which said longitudinal rod (2) extends with clearance,
**in that** the first connection members (12) are mounted onto said fastening means (10) in a removable manner, and
**in that** the connection members (12) are in snap-fastened onto the fastener means (10) and include lateral tabs (36) that terminate in snap-fastener means for engaging in complementary means (40, 41) of the fastener means (10, 62).

2. A system according to claim 1, **characterized in that** in said second connection member (12), the rod is prevented from pivoting about an axis perpendicular to the axis of the rod and to the above-mentioned transverse axis (X).

3. A system according to any one of claims 1 and 2, **characterized in that** each connection members (12) includes positioning and centering means (28, 32) for positioning and centering it on a socket (14) of fastener means (10, 62).

4. A system according to any preceding claim, **characterized in that** the rod (2) is made of a biocompatible plastics material such as PEEK.

5. A system according to any one of claims 1 to 4, **characterized in that** the rod (2) is, at least in part, of section that is not circular, and **in that** each connection member (12) includes a passage of corresponding section through which the rod passes with clearance, thereby opposing turning of said rod about its axis.

6. A system according to any one of claims 1 to 5, **characterized in that** it comprises two parallel longitudinal flexible rods (2) connected to means (10) for fastening on the vertebrae and united with each other by transverse connections (76) fastened at their ends on connection members (12) mounted on the flexible rods.

7. A system according to any preceding claim, **characterized in that** the means for fastening on the vertebrae comprise pedicular screws (10) or hooks (42) for clamping collars made of a flexible and biocompatible material such as PEEK.

8. A system according to any preceding claim, **characterized in that** the blocking means comprise a screw (54) that is screwed in a tapped hole of said second connection member (12)
